# EUROPEAN PATENT APPLICATION

(11) **EP 4 067 362 A1**
(43) Date of publication of application: **05.10.2022**
(21) Application number: 22165764.6
(22) Date of filing: 31.03.2022
(51) Int. Cl.: C07D 495/10, C09K 9/00, C09K 11/00

(54) **PHOTOCHROMIC COMPOUND, PHOTOCHROMIC ARTICLE AND EYEGLASSES**

(30) Priority: 31.03.2021 JP 2021062096; 24.02.2022 JP 2022026473
(71) Applicant: Hoya Lens Thailand Ltd., Pathumthani 12130 (TH)
(72) Inventor: ARAI, Yuko, Tokyo (JP); KOBAYASHI, Kei, Tokyo (JP); NUSHI, Yusuke, Tokyo (JP); HERON, Mark, Huddersfield (GB); GABBUTT, Christopher, Huddersfield (GB); CARVALHO COUTO DE AZEVEDO, Orlando Delfim, Huddersfield (GB)
(74) Representative: Beckmann, Claus

(57) **Abstract**

The present application relates to photochromic compounds represented by general formula (1):

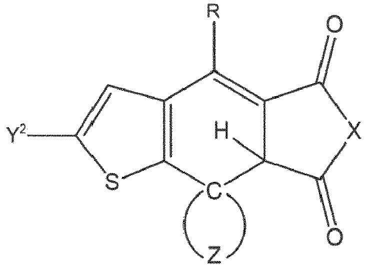

The application also relates to uses of said compounds in articles.

## Description

### TECHNICAL FIELD

The present invention relates to a photochromic compound, a photochromic article and eyeglasses.

### BACKGROUND

A photochromic compound is a compound having the property (photochromic property) of developing color under irradiation with light of the wavelength range having photoresponsive properties and fading the color under a non-irradiation state. Examples of articles (photochromic articles) provided with the photochromic property by a photochromic compound may include optical articles such as spectacle lenses.

In recent years, a fulgide compound is suggested as a compound to give the photochromic property to photochromic articles (for example, see PTL1).

PTL1: Japanese Patent Application Publication No. H07-2824

### SUMMARY OF THE INVENTION

Generally, fulgide compounds are said to be photochromic compounds that are likely to have good durability of photochromic performance. If the performance of such a fulgide compound as a photochromic compound can be further improved, a photochromic compound useful for producing photochromic articles can be provided.

One aspect of the present invention can achieve further improvement of the performance of a fulgide compound as a photochromic compound.

As a result of intensive study, the present inventors have newly found that a fulgide compound represented by the following general formula (1) is superior in photochromic performance.

That is, one aspect of the present invention relates to a photochromic compound represented by the following general formula (1).

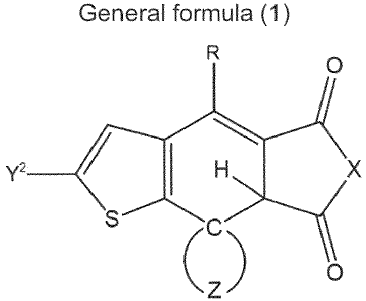

In the general formula (1),
X denotes an oxygen atom, or a nitrogen atom unsubstituted or substituted by a substituent selected from the following Y¹ group:
Y¹ group: -R¹, -A¹(B¹)ₗ(A²)ₘ(B²)ₙR², -A³A⁴, -A⁵R³
R¹ denotes a cyano group, an alkyl group or an aryl group which may each be substituted,
R² denotes an alkyl group, a naphthyl group, or a naphthyl alkyl group which may each be substituted,
R³ denotes a halogen atom, a cyano group, or a nitro group,
A¹, A², A³, and A⁵ each independently denote an alkylene group, an alkylidene group, a cycloalkylene group, or an alkylcycloalkane-diyl group which may each be substituted,
A⁴ denotes a naphthyl group which may be substituted,
B¹ and B² each independently denote a divalent group selected from the following group: 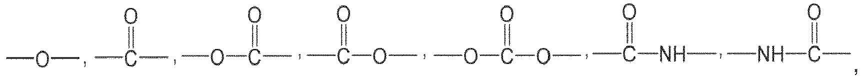
I, m, and n are each independently 0 or 1, provided that n is 0 when m is 0,
Y² denotes a hydrogen atom, or a substituent selected from the above Y¹ group,
R denotes a hydrogen atom, a trifluoromethyl group, or a cyclopropyl group which may be substituted, and 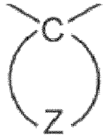
denotes a norbornylidene group, a bicyclo[3.3.1]nonylidene group, or an adamantylidene group which may each be substituted.

According to one aspect of the present invention, a fulgide compound that exhibits excellent photochromic performance can be provided.

### DESCRIPTION OF THE EMBODIMENTS

### Photochromic Compound

As one example, a photochromic compound undergoes an excited state by being irradiated with light such as sunlight and is structurally converted into a colored body. The structure after structural conversion via irradiation with light can be referred to as a "colored body". In contrast, the structure before irradiation with light can be referred to as a "colorless body". The term "colorless" in the "colorless body" does not limitedly mean a complete colorless state and encompasses a state where the color is pale compared to the colored body. The structure of the general formula (1) is a structure of a colorless body. The photochromic compound represented by the general formula (1) has a colorless body with a ring-closed structure.

The photochromic compound represented by the general formula (1) is hereinafter described in more detail. In the present invention and the present description, the photochromic compound represented by the general formula (1) encompasses geometrical isomers of structures indicated as the general formula (1).

In the general formula (1), X denotes an oxygen atom, or a nitrogen atom unsubstituted or substituted with a substituent selected from the following Y¹ group.
Y¹ group: -R¹, -A¹(B¹)ₗ(A²)ₘ(B²)ₙR², -A³A⁴, -A⁵R³

R¹ denotes a cyano group, an alkyl group which may be substituted, or an aryl group which may be substituted.

Examples of alkyl groups represented by R¹ may include a methyl group, an ethyl group, a propyl group, a n-, iso-, or ter-butyl group, a pentyl group, a hexyl group, an octyl group, a decyl group, and the like. Among these, an alkyl group with C (i.e., carbon number) 1-20 is preferable, and an alkyl group with C1-10 is more preferable.

Examples of aryl groups represented by R¹ may include an aryl group with C6-10 such as a phenyl group, a biphenyl group, a tolyl group, or a naphthyl group.

R² denotes an alkyl group which may be substituted, a naphthyl group which may be substituted, and a naphthylalkyl group which may be substituted.

Regarding the alkyl group represented by R², the former description about the alkyl group represented by R¹ can be referred to. The carbon number in an alkyl group represented by R² is not particularly limited and may preferably be 1 to 10.

Examples of the naphthylalkyl group represented by R² may include a naphthylmethyl group, a naphthylethyl group, a naphthylpropyl group, a naphthylbutyl group, or the like. The carbon number in an alkyl group of a naphthylalkyl group is preferably 1 to 4.

R³ represents a halogen atom, a cyano group, or a nitro group. Examples of halogen atoms represented by R³ may include a fluorine atom, a chlorine atom, a bromine atom, or the like.

A¹, A², A³, and A⁵ each independently represent an alkylene group which may be substituted, an alkylidene group which may be substituted, a cycloalkylene group which may be substituted, or an alkylcycloalkane-diyl group which may be substituted. Specific examples of these may include alkylene groups with C1-10 such as a methylene group, an ethylene group, a propylene group, a butylene group, a trimethylene group, a tetramethylene group, or a 2,2-dimethyltrimethylene group; alkylidene groups with C2-10 such as an ethylidene group, a propylidene group, or an isopropylidene group; and cycloalkylene groups with C3-10 such as a cyclohexylene group; alkylcycloalkane-diyl groups with C6-10 such as a 2-methylcyclohexane-α,1-diyl group represented by the following formula: 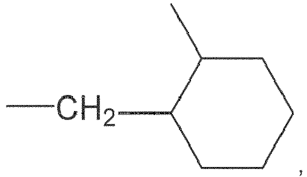 a 4-methylcyclohexane-α,1-diyl group represented by the following formula: 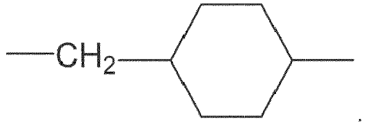 It is preferable that A¹, A², A³, and A⁵ are each independently an alkylene group with C1-6, an alkylidene group with C2-6, a cycloalkylene group with C3-6, or an alkylcycloalkane-diyl group with C6-7.

B¹ and B² each independently denote any one of the divalent groups selected from the following group:

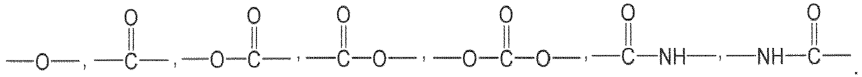

I, m, and n are each independently 0 or 1, provided that n is 0 if m is 0.

Each of the groups described above is unsubstituted in one embodiment and substituted in other one embodiment. The substituent is not particularly limited. When a group has a substituent or substituents, the carbon number described above about each group shall refer to the carbon number in the part that does not include a substituent. For example, the alkyl group may be substituted with one to three atoms and/or groups selected from the group consisting of halogen atoms, cyano groups, and nitro groups. The naphthyl group or the naphthylalkyl group may be substituted with one to three atoms and/or groups selected from the group consisting of halogen atoms, cyano groups, nitro groups, alkylamino groups with C1-3, alkyl groups with C1-3, or alkoxy groups with C1-3.

A⁴ denotes a naphthyl group which may be substituted. The naphthyl group represented by A⁴ is unsubstituted in one embodiment and substituted in other one embodiment. The type of such a substituent is not particularly limited. For example, the naphthyl group represented by A⁴ may be substituted with one to three atoms and/or groups selected from the group consisting of halogen atoms, cyano groups, and nitro groups, alkylamino groups with C1-3, alkyl groups with C1-3, and alkoxy groups with C1-3.

Y² denotes a hydrogen atom, or a substituent selected from the Y¹ group described above. Y² is preferably a hydrogen atom in one embodiment. In other one embodiment, Y² is preferably -R¹ in the Y¹ group, more preferably an aryl group which may be substituted, further preferably an aryl group which is substituted, and is still more preferably represented by -R¹¹-R¹², wherein -R¹¹- is selected from the group consisting of the following group, and -R¹² is -(CH₂)ₙCH₃, where n is an integer within the range of 3 to 10.

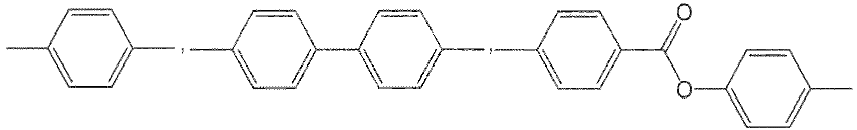

R denotes a hydrogen atom, a trifluoromethyl group, or a cyclopropyl group which may be substituted. The cyclopropyl group represented by R is unsubstituted in one embodiment and substituted in other one embodiment. As such a substituent, a substituent selected from the above Y¹ group may be exemplified. In one embodiment, R preferably denotes a cyclopropyl group which may be substituted and more preferably denotes an unsubstituted cyclopropyl group.

In the general formula (1), 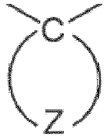 denotes a norbornylidene group which may be substituted, a bicyclo[3.3.1]nonylidene group which may be substituted, or an adamantylidene group which may be substituted.

As the norbornylidene group, the following 7-norbornylidene group is preferable.

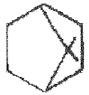

As the bicyclo[3.3.1]nonylidene group, the following bicyclo[3.3.1]9-nonylidene group is preferable.

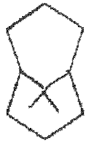

As the adamantylidene group, the following 2-adamantylidene group is preferable.

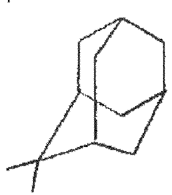

The above structures of the 7-norbornylidene group, bicyclo[3.3.1]nonylidene group, and 2-adamantylidene group are all structures with no substituent.

In the general formula (1), a norbornylidene group, a bicyclo[3.3.1]nonylidene group, and an adamantylidene group represented by 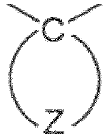 are all unsubstituted in one embodiment and are substituted in other one embodiment. When a group has a substituent or substituents, the number of substituents may be one, or two or more. Examples of the substituent may include a hydroxy group; alkylamino groups with C1-4 such as a methylamino group and a diethylamino group; alkoxy group with C1-4 such as a methoxy group, an ethoxy group, and a tert-butoxy group; aralkoxy groups with C7-15 such as a benzyloxy group; aryloxy groups with C6-14 such as a phenoxy group and a 1-naphthoxy group; alkyl groups with C1-4 such as a methyl group, an ethyl group, and a t-butyl group; halogen atoms such as a fluorine atom, a chlorine atom, and a bromine atom; a cyano group; a carboxy group; alkoxycarbonyl groups with C2-10 such as an ethoxycarbonyl group; halogen-substituted alkyl groups with C1-2 such as a trifluoromethyl group; a nitro group; aryl groups with C6-10 such as a phenyl group and a toluyl group, aralkyl groups with C7-9 such as a phenylethyl group and a phenylpropyl group, and the like. Preferable examples of these substituents may include halogen atoms, a hydroxy group, alkyl groups with C1-4, alkoxy groups with C1-4, alkoxycarbonyl groups with C2-10, aralkyl groups with C7-9, or aryl groups with C6-10.

The photochromic compound represented by the general formula (1) can be used for preparing an article (photochromic article) with the photochromic property. The following compounds may be listed as specific examples of such a compound. However, the present invention is not limited to the listed compounds. The following structures are colorless structures.

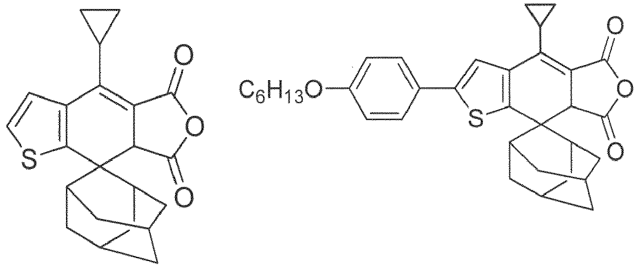

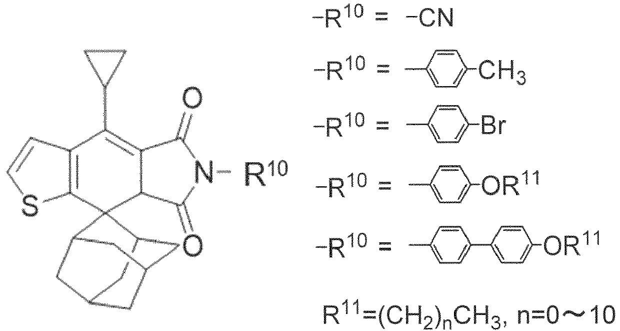

The synthetic methods of the photochromic compound represented by the general formula (1) are not particularly limited. For example, paragraphs [0068] to [0099] and the examples of Japanese Patent Application Publication No. H07-2824 can be referred to for the synthetic method thereof. Also, the identification methods of the compound are not particularly limited. For example, paragraphs [0063] to [0066] of Japanese Patent Application Publication No. H07-2824 can be referred to for the identification method. The disclosure in the section of Examples, which will be described below, can also be referred to for the synthetic method thereof.

### Photochromic Article

One aspect of the present invention relates to a photochromic article including the photochromic compound represented by the general formula (1).

In the present invention and the present description, a "photochromic article" shall mean an article including at least one photochromic compound. The photochromic compound represented by the general formula (1) may be included in the photochromic layer of a photochromic article and/or may be included in the substrate of a photochromic article. In an embodiment, such a photochromic article may include only one compound represented by the general formula (1), and in other one embodiment, such a photochromic article may include two or more compounds represented by the general formula (1). In one embodiment, the photochromic article may contain at least one compound represented by the general formula (1) and at least one other photochromic compound. Examples of the other photochromic compounds may include azobenzenes, spiropyrans, spirooxazines, naphthopyrans, indenonaphthopyrans, phenanthropyrans, hexaallylbisimidazoles, donor-acceptor Stenhouse adducts (DASA), salicylideneanilines, dihydropyrenes, anthracene dimers, fulgides, diarylethenes, phenoxy naphthacenequinones, stilbenes, and the like.

The photochromic article may include a substrate selected depending on the type of the photochromic article. Examples of the substrate may include plastic lens substrate or glass lens substrate for spectacle lens substrate. For example, the glass lens substrate may be a lens substrate made of inorganic glass. Examples of a plastic lens substrate may include a styrene resin, including a (meth)acrylate resin, a polycarbonate resin, allylic resins such as a diethylene glycol bis(allyl carbonate) resin (CR-39), a vinyl resin, a polyester resin, a polyether resin, a urethane resin obtained by a reaction between an isocyanate compound and a hydroxy compounds such as diethylene glycol, a thiourethane resin obtained by a reaction between an isocyanate compound and a polythiol compound, a cured product (which is generally called a transparent resin) of a curable composition containing a (thio)epoxy compound having at least one disulfide bond in a molecule, and the like. As a lens substrate, an undyed substrate (colorless lens) may be used, or a dyed substrate (dyed lens) may be used. For example, the refractive index of the lens substrate may be around 1.60 to 1.75. However, the refractive index of the lens substrate is not limited to the above range and may be within the range or may be over or below the range. The refractive index shall herein mean a refractive index for the light of wavelength 500 nm. The lens substrate may be a lens with refractive power (so-called prescription lens) or may be a lens without refractive power (so-called non-prescription lens).

For example, a substrate can be prepared as a curable product of a curable composition containing at least one photochromic compound represented by the general formula (1) and at least one polymerizable compound by molding the curable composition in a known molding method. The curable composition may further contain at least one other photochromic compound, for example, as those listed earlier. Curing treatment may be irradiation with light and/or heat treatment. A polymerizable compound is a compound with a polymerizable group, and a curable composition can be cured as the progress of the polymerization reaction of the polymerizable compound to form a cured product. The curable composition may contain one or more additives (for example, a polymerization initiator).

The spectacle lens may be various lenses such as a unifocal lens, a multifocal lens, and a progressive power lens. The lens type is determined depending on the surface shapes of both sides of the lens substrate. Also, the lens substrate surface may be any of convex, concave, or flat. A normal lens substrate and spectacle lens have a convex object-side surface and a concave eyeball-side surface. However, the lens substrate and the spectacle lens are not limited to these. The photochromic layer may be normally disposed on the surface of the object side of the lens substrate but may be disposed on the surface of the eyeball side.

The photochromic layer may be a layer disposed directly on the surface of the substrate or may be disposed indirectly on the surface via at least one other layer. For example, the photochromic layer may be a cured layer obtained by curing a curable composition containing a photochromic compound represented by the general formula (1). The curable composition may further contain at least one other photochromic compound, for example, as those listed earlier. For example, a cured layer containing the photochromic compound can be formed by directly applying the curable composition to the surface of a substrate or applying the curable composition on a layer provided on a substrate and subsequently subjecting the applied curable composition to curing treatment. For a coating method, known coating methods such as spin coating and dip coating may be adopted. Curing treatment may be irradiation with light and/or heat treatment. The curable composition may contain a polymerizable compound and may further contain one or more additives (for example, a polymerization initiator). The curable composition can be cured as the progress of the polymerization reaction of the polymerizable compound to form a cured layer.

### Polymerizable Compound

In the present invention and the present description, a polymerizable compound shall refer to a compound with one or more polymerizable group in one molecule, and a "polymerizable group" shall refer to a reactive group that can undergo polymerization reaction. Examples of the polymerizable group may include an acryloyl group, a methacryloyl group, a vinyl group, a vinyl ether group, an epoxy group, a thiol group, an oxetane group, a hydroxy group, a carboxy group, an amino group, an isocyanate group, and the like.

As examples of polymerizable compounds available for forming the above substrate and the above photochromic layer, the following compounds may be exemplified.

### Episulfide-based Compound

An episulfide-based compound is a compound with two or more episulfide groups in one molecule. An episulfide group is a polymerizable group that can undergo ring-opening polymerization. Specific examples of episulfide compounds may include bis(1,2-epithioethyl) sulfide, bis(1,2-epithioethyl) disulfide, bis(2,3-epithiopropyl) sulfide, bis(2,3-epithiopropylthio)methane, bis(2,3-epithiopropyl) disulfide, bis(2,3-epithiopropyldithio)methane, bis(2,3-epithiopropyldithio)ethane, bis(6,7-epithio-3,4-dithiaheptyl) sulfide, bis(6,7-epithio-3,4-dithiaheptyl) disulfide, 1,4-dithiane-2,5-bis(2,3-epithiopropyldithiomethyl), 1,3-bis(2,3-epithiopropyldithiomethyl)benzene, 1,6-bis(2,3-epithiopropyldithiomethyl)-2-(2,3-epithiopropyldithioethylthio)-4-thiahexane, 1,2,3-tris(2,3-epithiopropyldithio)propane, 1,1,1,1-tetrakis(2,3-epithiopropyldithiomethyl)methane, 1,3-bis(2,3-epithiopropyldithio)-2-thiapropane, 1,4-bis(2,3-epithiopropytdithio)-2,3-dithiabutane, 1,1,1-tris(2,3-epithiopropyidithio)methane, 1,1,1-tris(2,3-epithiopropyldithiomethylthio)methane, 1,1,2,2-tetrakis(2,3-epithiopropyldithio)ethane, 1,1,2,2-tetrakis(2,3-epithiopropyldithiomethylthio)ethane, 1,1,3,3-tetrakis(2,3-epithiopropyidithio)propane, 1,1,3,3-tetrakis(2,3-epithiopropyldithiomethylthio)propane, 2-[1,1-bis(2,3-epithiopropyldithio)methyl]-1,3-dithietane, 2-[1,1-bis(2,3-epithiopropyldithiomethylthio)methyl]-1,3-dithietane, and the like.

### Thietanyl Compound

The thietanyl compound is a thietane compound having two or more thietanyl groups in one molecule. A thietanyl group is a polymerizable group that can undergo ring-opening polymerization. Some thietanyl compounds have an episulfide group together with a plurality of thietanyl groups. Such compounds are listed in the examples of the episulfide compounds described above. Other thietanyl compounds include metal-containing thietane compounds containing metal atoms in a molecule and nonmetal-containing thietane compounds containing no metal.

Specific examples of the non-metal thietane compounds may include bis(3-thietanyl) disulfide, bis(3-thietanyl) sulfide, bis(3-thietanyl) trisulfide, bis(3-thietanyl) tetrasulfide, 1,4-bis(3-thietanyl)-1,3,4-trithiabutane, 1,5-bis(3-thietanyl)-1,2,4,5-tetrathiapentane, 1,6-bis(3-thietanyl)-1,3,4,6-tetrathiahexane, 1,6-bis(3-thietanyl)-1,3,5,6-tetrathiahexane, 1,7-bis(3-thietanyl)-1,2,4,5,7-pentathiaheptane, 1,7-bis(3-thietanylthio)-1,2,4,6,7-pentathiaheptane, 1,1-bis(3-thietanylthio)methane, 1,2-bis(3-thietanylthio)ethane, 1,2,3-tris(3-thietanylthio)propane, 1,8-bis(3-thietanylthio)-4-(3-thietanylthiomethyl)-3,6-dithiaoctane, 1,11-bis(3-thietanylthio)-4,8-bis(3-thietanylthiomethyl)-3,6,9-trithiaundecane, 1,11-bis(3-thietanylthio)-4,7-bis(3-thietanylthiomethyl)-3,6,9-trithiaundecane, 1,11-bis(3-thietanylthio)-5,7-bis(3-thietanylthiomethyl)-3,6,9-trithiaundecane, 2,5-bis(3-thietanylthiomethyl)-1,4-dithiane, 2,5-bis[[2-(3-thietanylthio)ethyl]thiomethyl]-1,4-dithiane, 2,5-bis(3-thietanylthiomethyl)-2,5-dimethyl-1,4-dithiane, bisthietanyl sulfide, bis(thietanylthio)methane, 3-[<(thietanylthio)methylthio>methylthio]thietane, bisthietanyl disulfide, bisthietanyl trisulfide, bisthietanyl tetrasulfide, bisthietanyl pentasulfide, 1,4-bis(3-thietanyldithio)-2,3-dithiabutane, 1,1,1-tris(3-thietanyldithio)methane, 1,1,1-tris(3-thietanyldithiomethylthio)methane, 1,1,2,2-tetrakis(3-thietanyldithio)ethane, 1,1,2,2-tetrakis(3-thietanyldithiomethylthio)ethane, and the like.

Examples of the metal-containing thietane compounds may include compounds containing, in a molecule, group 14 atoms such as a Sn atom, a Si atom, a Ge atom, and a Pb atom, group 4 atoms such as a Zr atom and a Ti atom, group 13 atoms such as an Al atom, group 12 atoms such a Zn atom, and the like, as metal atoms. Specific examples may include alkylthio(thietanylthio)tins, bis(alkylthio)bis(thietanylthio)tins, alkylthio(alkylthio)bis(thietanylthio)tins, bis(thietanylthio) cyclic dithiotin compounds, alkyl(thietanylthio)tin compounds, and the like.

Specific examples of alkylthio(thietanylthio)tins may include methylthiotris(thietanylthio)tin, ethylthiotris(thietanylthio)tin, propylthiotris(thietanylthio)tin, isopropylthiotris(thietanylthio)tin, and the like.

Specific examples of bis(alkylthio)bis(thietanylthio)tins may include bis(methylthio)bis(thietanylthio)tin, bis(ethylthio)bis(thietanylthio)tin, bis(propylthio)bis(thietanylthio)tin, bis(isopropylthio)bis(thietanylthio)tin, and the like.

Specific examples of alkylthio(alkylthio)bis(thietanylthio)tins may include ethylthio(methylthio)bis(thietanylthio)tin, methylthio(propylthio)bis(thietanylthio)tin, isopropylthio(methylthio)bis(thietanylthio)tin, ethylthio(propylthio)bis(thietanylthio)tin, ethylthio(isopropylthio)bis(thietanylthio)tin, isopropylthio(propylthio)bis(thietanylthio)tin, and the like.

Specific example of the bis(thietanylthio) cyclic dithiotin compounds may include bis(thietanylthio)dithiastannetane, bis(thietanylthio)dithiastannolane, bis(thietanylthio)dithiastanninane, bis(thietanylthio)trithiastannocane, and the like.

Specific examples of the alkyl(thietanylthio)tin compounds may include methyltris(thietanylthio)tin, dimethylbis(thietanylthio)tin, butyltris(thietanylthio)tin, tetrakis(thietanylthio)tin, tetrakis(thietanylthio)germanium, tris(thietanylthio)bismuth, and the like.

### Polyamine Compound

A polyamine compound is a compound having two or more NH₂ groups in one molecule, can form a urea linkage by reaction with a polyisocyanate, and can form a thiourea linkage by reaction with a polyisothiocyanate. Specific examples of the polyamine compound may include ethylenediamine, hexamethylenediamine, isophoronediamine, nonamethylenediamine, undecamethylenediamine, dodecamethylenediamine, meta-xylenediamine, 1,3-propanediamine, putrescine, 2-(2-aminoethylamino)ethanol, diethylenetriamine, p-phenylenediamine, m-phenylenediamine, melamine, 1,3,5-benzenetriamine, and the like.

### Epoxy-based Compound

An epoxy-based compound is a compound with an epoxy group in a molecule. An epoxy group is a polymerizable group that can undergo ring-opening polymerization. Generally, epoxy-based compounds are classified into aliphatic epoxy compounds, alicyclic epoxy compounds, and aromatic epoxy compounds.

Specific examples of the aliphatic epoxy compounds may include ethylene oxide, 2-ethyloxirane, butyl glycidyl ether, phenyl glycidyl ether, 2,2'-methylenebisoxirane, 1,6-hexanediol diglycidyl ether, ethylene glycol diglycidyl ether, diethylene glycol diglycidyl ether, triethylene glycol diglycidyl ether, tetraethylene glycol diglycidyl ether, nonaethylene glycol diglycidyl ether, propylene glycol diglycidyl ether, dipropylene glycol diglycidyl ether, tripropylene glycol diglycidyl ether, tetrapropylene glycol diglycidyl ether, nonapropylene glycol diglycidyl ether, neopentylglycol diglycidyl ether, trimethylolpropane triglycidyl ether, glycerol triglycidyl ether, diglycerol tetraglycidyl ether, pentaerythritol tetraglycidyl ether, a triglycidyl ether of tris(2-hydroxyethyl) isocyanurate, and the like.

Specific examples of the alicyclic epoxy compounds may include isophorone diol diglycidyl ether, bis-2,2-hydroxycyclohexylpropane diglycidyl ether, and the like.

Specific examples of the aromatic epoxy compounds may include resorcindiglycidylether, bisphenol A diglycidyl ether, bisphenol F diglycidyl ether, bisphenol S diglycidyl ether, diglycidyl orthophthalate, phenolic novolac polyglycidyl ether, cresol novolac polyglycidyl ether, and the like.

Besides the above, an epoxy-based compound with a sulfur atom together with an epoxy group in a molecule can be used. Such sulfur atom-containing epoxy-based compounds include chain aliphatic types and cyclic aliphatic types.

Specific examples of chain aliphatic sulfur atom-containing epoxy-based compound may include bis(2,3-epoxypropyl) sulfide, bis(2,3-epoxypropyl) disulfide, bis(2,3-epoxypropylthio)methane, 1,2-bis(2,3-epoxypropylthio)ethane, 1,2-bis(2,3-epoxypropylthio)propane, 1,3-bis(2,3-epoxypropylthio)propane, 1,3-bis(2,3-epoxypropylthio)-2-methylpropane, 1,4-bis(2,3-epoxypropylthio)butane, 1,4-bis(2,3-epoxypropylthio)-2-methylbutane, 1,3-bis(2,3-epoxypropylthio)butane, 1,5-bis(2,3-epoxypropylthio)pentane, 1,5-bis(2,3-epoxypropylthio)-2-methylpentane, 1,5-bis(2,3-epoxypropylthio)-3-thiapentane, 1,6-bis(2,3-epoxypropylthio)hexane, 1,6-bis(2,3-epoxypropylthio)-2-methylhexane, 3,8-bis(2,3-epoxypropylthio)-3,6-dithiaoctane, 1,2,3-tris(2,3-epoxypropylthio)propane, 2,2-bis(2,3-epoxypropylthio)-1,3-bis(2,3-epoxypropylthiomethyl)propane, 2,2-bis(2,3-epoxypropylthiomethyl)-1-(2,3-epoxy propylthio)butane, and the like.

Specific examples of the cyclic aliphatic sulfur atom-containing epoxy-based compound may include 1,3-bis(2,3-epoxypropylthio)cyclohexane, 1,4-bis(2,3-epoxypropylthio)cyclohexane, 1,3-bis(2,3-epoxypropylthiomethyl)cyclohexane, 1,4-bis(2,3-epoxypropylthiomethyl)cyclohexane, 2,5-bis(2,3-epoxypropyithiomethyl)-1,4-dithiane, 2,5-bis[<2-(2,3-epoxypropylthio)ethyl>thiomethyl]-1,4-dithiane, 2,5-bis(2,3-epoxypropylthiomethyl)-2,5-dimethyl-1,4-dithiane, and the like.

### Compound with Radically Polymerizable Group

A radically polymerizable group is a group being capable of radical polymerization. Examples of the radically polymerizable groups may include an acryloyl group, a methacryloyl group, an allyl group, a vinyl group, and the like.

In the following, a compound with one or more polymerizable groups selected from the group consisting of an acryloyl group and a methacryloyl group is referred to as a "(meth)acrylate compound". Specific examples of the (meth)acrylate compounds may include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, tripropylene glycol di(meth)acrylate, polypropylene glycol di(meth)acrylate, neopentyl glycol di(meth)acrylate, ethylene glycol bisglycidyl (meth)acrylate, bisphenol A di(meth)acrylate, 2,2-bis(4-(meth)acryloxyethoxyphenyl)propane, 2,2-bis(4-(meth)acryloxydiethoxyphenyl)propane, 2,2-bis(4-(meth)acryloxyethoxyphenyl)propane, 2,2-bis(3,5-dibromo-4-(meth)acryloyloxyethoxyphenyl)propane, 2,2-bis(4-(meth)acryloyloxydipropoxyphenyl) propane, bisphenol F di(meth)acrylate, 1,1- bis(4-(meth)acryloxyethoxyphenyl)methane, 1,1-bis(4-(meth)acryloxydiethoxyphenyl)methane, dimethyloltricyclodecane di(meth)acrylate, trimethylolpropane tri(meth)acrylate, ditrimethylolpropane tetra(meth)acrylate, glycerol di(meth)acrylate, pentaerythritol tri(meth)acrylate, pentaerythritol tetra(meth)acrylate, methyl thio(meth)acrylate, phenyl thio(meth)acrylate, benzyl thio(meth)acrylate, xylylenedithiol di(meth)acrylate, mercaptomethyl sulfide di(meth)acrylate, bifunctional urethane (meth)acrylates, and the like.

Specific examples of the compounds with one or more allyl groups (allyl compounds) may include allyl glycidyl ether, diallyl phthalate, diallyl terephthalate, diallyl isophthalate, diallyl carbonate, diethylene glycol bis(allyl carbonate), methoxypolyethylene glycol allyl ether, polyethylene glycol allyl ether, methoxypolyethylene glycol-polypropylene glycol allyl ether, butoxypolyethylene glycol-polypropylene glycol allyl ether, methacryloyloxypolyethylene glycol-polypropylene glycol allyl ether, phenoxypolyethylene glycol allyl ether, methacryloyloxypolyethylene glycol allyl ether, and the like.

Examples of the compounds with one or more vinyl groups, (vinyl compounds) may include α-methylstyrene, α-methylstyrene dimer, styrene, chlorostyrene, methylstyrene, bromostyrene, dibromostyrene, divinylbenzene, 3,9-divinylspirobi(m-dioxane), and the like.

The above photochromic article may include, in any position, at least one layer known as a functional layer for optical articles, such as a protective layer for improving durability, an anti-reflective layer, a water-repellent or hydrophilic antifouling layer, an anti-fogging layer, a primer layer for improving interlaminar adhesion.

An embodiment of the photochromic article is an optical article, and examples of the optical article include spectacle lenses, lenses for goggles, visor (sunshade) parts of sun visors, shield members of helmets, and the like. For example, an optical article with anti-glare functions can be obtained by coating the curable composition on a substrate for these optical articles and subjecting the coated composition to curing treatment to form a photochromic layer.

### Eyeglasses

One aspect of the present invention relates to eyeglasses with spectacle lenses, which is one embodiment of the photochromic article. More detailed information about the spectacle lenses of these eyeglasses was as described earlier. The eyeglasses provided with such spectacle lenses can exhibit an anti-glare effect outdoors, like sunglasses, by the photochromic compound being irradiated with the sunlight and developing a color, and in contrast, can recover the transparency when a wearer goes back indoors by the photochromic compound fading the color. A known technique can be applied to the structure of frames or the like of the above eyeglasses.

### EXAMPLES

The present invention will be further described with reference to Examples.

In the following, at least one of a nuclear magnetic resonance (NMR) apparatus, an infrared spectrometer (IR), and a melting point measurement was used for identification of molecular structures.

### Example 1

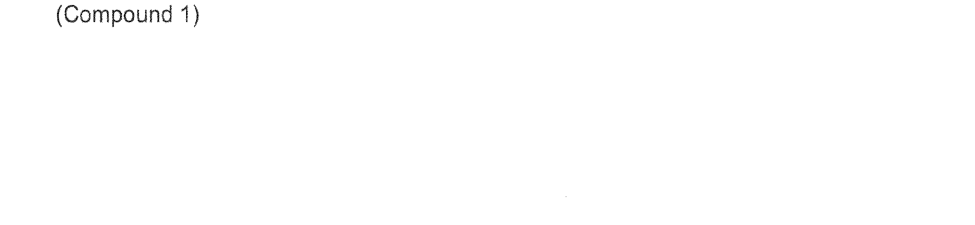

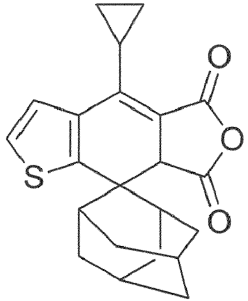

### Synthesis of Compound 1

A compound 1 ((1r,3r,5r,7r)-4'-cyclopropyl-5'H-spiro[adamantane-2,8'-thieno[2,3-f]isobenzofuran]-5',7'(7a'H)dione) was synthesized according to the following synthetic scheme.

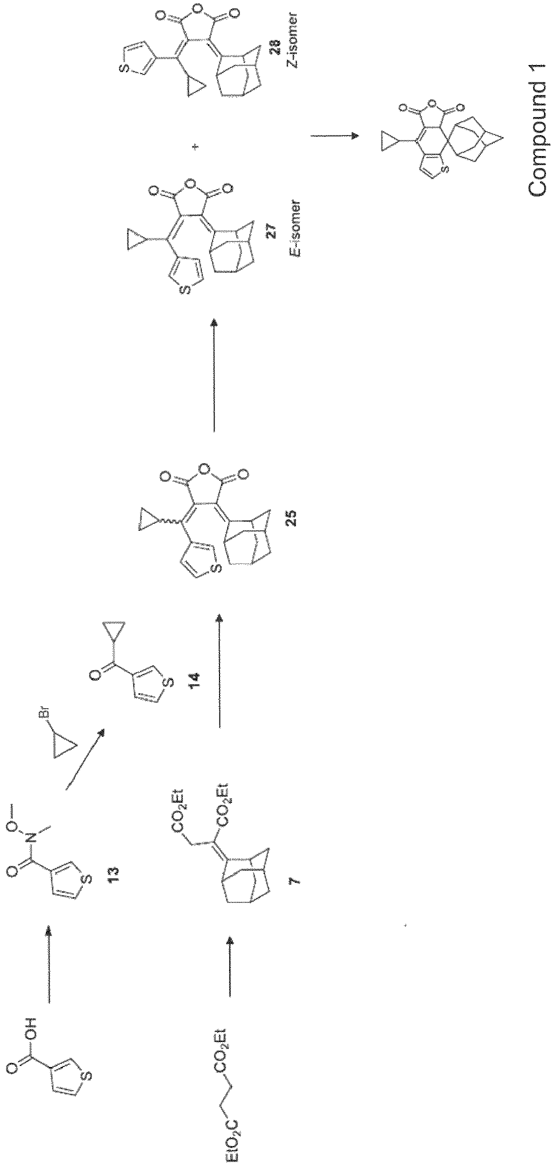

A compound 7 was synthesized according to the above synthetic scheme by the methods disclosed in C. J. Thomas, M. A. Wolak, R. R. Birge and W. J. Lees. J. Org. Chem., 2001, 66, 1914-1918.

A compound 14 was synthesized according to the above synthetic scheme by a method disclosed in WO 2011/042918.

According to the following method, a compound 1 was obtained from a mixture of a compound 27 and a compound 28.

A mixture of compound 27 and compound 28 (2.00 g, 5.46 mmol) were dissolved in degassed toluene (10.0 mL) at 80 °C and irradiated with UV radiation (365 nm) for 4 days under a flow of N2. Afterwards, crystallization from hot toluene allowed removal of compound 27. Flash column chromatography (Aldrich silica gel 60 Å 70-230 mesh 63-200 µm; eluent: toluene) afforded compound 1 as a pale yellow powder (0.74 g, 37%).

Compound 1: ¹H NMR (400 MHz, CDCl₃) δH 0.91-0.96 (1H, m, CH), 1.17-1.26 (3H, m, CH), 1.54-1.97 (10H, m, CH), 2.317-2.322 (1H, m, CH), 2.53 (1H, app. s, CH), 2.83-2.89 (1H, m, CH), 3.03-3.07 (1H, m, CH), 3.31-3.36 (1H, m, CH), 3.93 (1H, d, J = 1.1 Hz, CH), 7.09 (1H, d, J = 5.4 Hz, Ar-H), 7.19 (1H, d, J = 5.4 Hz, Ar-H) ppm.

### Evaluation of Photochromic Performance

The above compound 1 was dissolved in chloroform that did not contain any stabilizer to prepare a solution of the compound 1 in chloroform.

The prepared solution was put in a 1-cm square quartz spectroscopic cell, and the cell was covered with a lid. The solution was then measured for luminous transmittance (Tini) in an unirradiated state and luminous transmittance (Tact) at a time when the color development was saturated after the irradiation with artificial sunlight for a certain period of time in an ultraviolet and visible spectrophotometer (UV-1900i, manufactured by Shimadzu Corporation, the measurement wavelength: 800 to 250 nm, measured at a pitch of 2 nm wavelength, fast mode). The measurement was carried out at room temperature (23°C). The concentration of the solution was adjusted so that the absorptivity of the first absorption wavelength (peak of the absorption strength observed at the longest wavelength) became 0.95 to 1.05. The dynamic range "(Tini) - (Tact)" can be used as an index of the degree of the color development of a colored body with respect to a colorless body, and it may be considered that as the value of the dynamic range is larger, the photochromic performance is better.

### Evaluation of Color Fading Speed

The color fading speed of a spectacle lens having a photochromic layer that contains the compound 1 at a concentration of 1 part by mass was evaluated by the following method. The concentration of the above compound represents the content of the photochromic compound in relation to 100 parts by mass of the total amount of polymerizable compounds in a curable composition used for forming a photochromic layer.

The surface of a photochromic layer of a spectacle lens was irradiated with light for 15 minutes (900 seconds) via an aero mass filter using a xenon lamp to develop the color of the photochromic compound in the photochromic layer. After that, the irradiation with light was stopped. The irradiation with light was carried out such that the irradiance and the tolerance of the irradiance were to be the values shown in table 1 as regulated in JIS T 7333:2005.

**Table 1**

| Wavelength range (nm) | Irradiance (W/m²) | Tolerance of irradiance (W/m²) |
|---|---|---|
| 300-340 | <2.5 | - |
| 340-380 | 5.6 | ±1.5 |
| 380-420 | 12 | ±3.0 |
| 420-460 | 12 | ±3.0 |
| 460-500 | 26 | ±2.6 |

A value determined by dividing the variation of the luminous transmittance during a certain period of time (30 seconds) after stopping the irradiation with light by the period of time is defined as a fading speed T. Table 2 shows the T values. The larger T value means a faster fading speed.

### Example 2

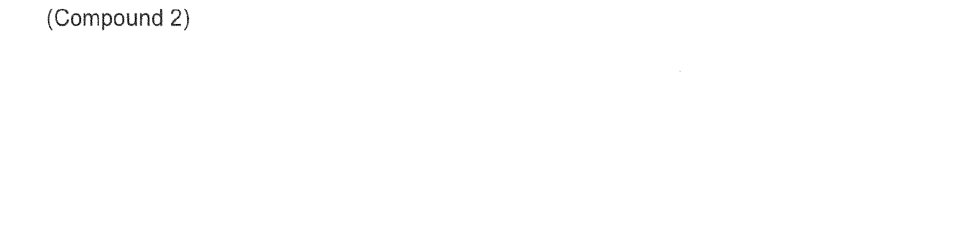

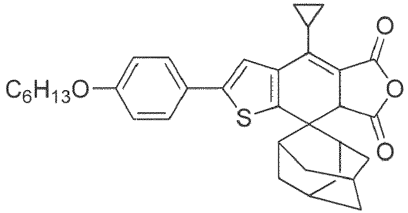

### Synthesis of Compound 2

A compound 2 ((1R,3R)-4'-cyclopropyl-2'-(4-(hexyloxy)phenyl-5'H-spiro[adamantane-2,8'-thieno[2,3-f]isobenzofuran]-5',7'(7a'H)-dione) was synthesized according to the following synthetic scheme.

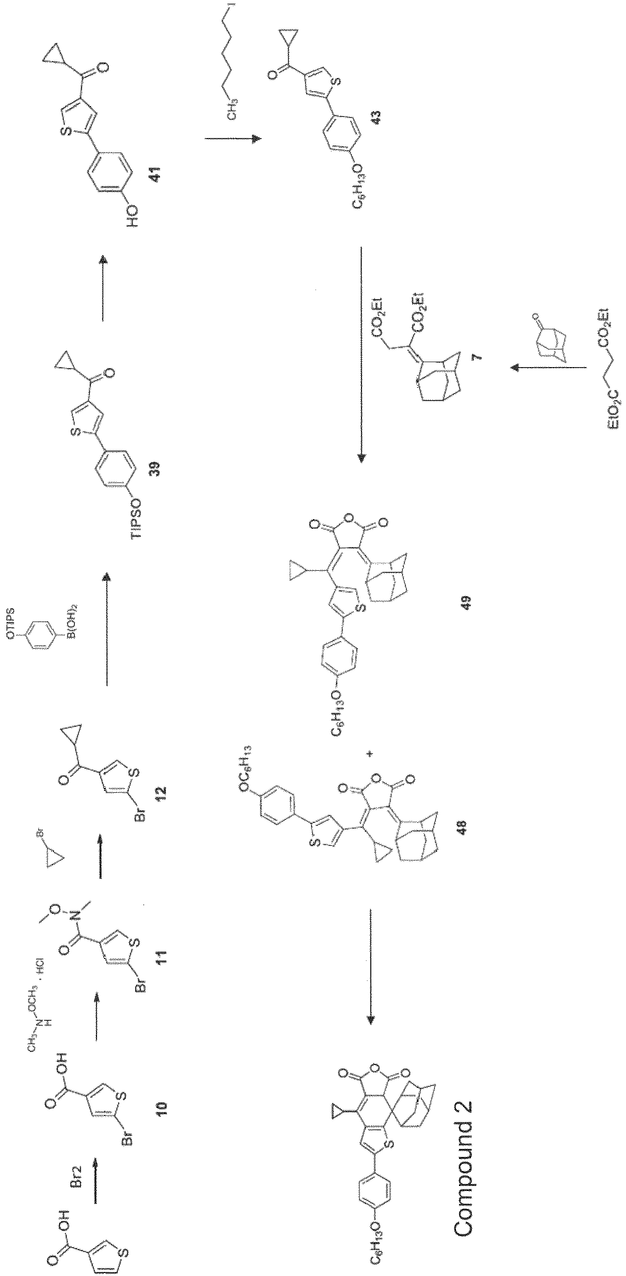

A compound 10 was synthesized according to the above synthetic scheme by a method disclosed in WO 2010/065383. Specifically, the compound 10 (5-bromothiophene-3-carboxylic acid) was synthesized by the following method.

Bromine (9 mL, 0.2 mol) in glacial acetic acid (190 mL) was added dropwise to a solution of thiophene-3-carboxylic acid (24.00 g, 0.1873 mol) in glacial acetic acid (190 mL). After stirring at room temperature for 90 min, the crude was poured into ice-water (1 L), a white precipitate was filtered, triturated with hot water (200 mL), filtered, rinsed with water (3 × 100 mL) and dried under reduced pressure. Crystallization from hot ethanol afforded the corresponding product as a white crystalline solid (15.45 g, 40%).

A compound 11 was synthesized according to the above synthetic scheme by a method disclosed in WO 2009/130193. Specifically, the compound 11 (5-bromo-N-methoxy-N-methylthiophene-3-carboxamide) was synthesized by the following method.

Thionyl chloride (25 mL, 345 mmol) was added to a cooled solution (ice/brine bath) of 5-bromothiophene-3-carboxylic acid (13.50 g, 65.20 mmol) in dichloromethane (450 mL). After stirring at room temperature for 30 min, the reaction mixture was refluxed for 3h30 (CaCl₂ drying tube fitted on top of the condenser). After cooling to room temperature, the crude was evaporated to dryness giving a yellow powder. *N,O-*dimethylhydroxylamine hydrochloride (7.00 g, 71.8 mmol) was added to the latter, and the solids suspended in dichloromethane (250 mL). Triethylamine (18.0 mL, 129 mmol) was added dropwise to the resulting mixture at 0 °C under N2. After stirring at room temperature for 3h, the crude was poured into water (200 mL), the phases separated and the aqueous phase extracted with dichloromethane (100 mL). The combined organic phases were washed with water (2 × 150 mL), dried with anhydrous Na2SO4, filtered and evaporated to dryness, affording the target product as an amber oil (12.59 g, 62%, Purity = 80%) that was used in the next step without further purification.

A compound 12 was synthesized according to the above synthetic scheme by a method disclosed in WO 2008/156601. Specifically, the compound 12 ((5-bromothiophene-3-yl)(cyclopropyl)methanone) was synthesized by the following method.

Magnesium turnings (0.55 g, 22.6 mmol) and a few crystals of molecular iodine were added to a dry three-neck 250 mL round-bottom flask (equipped with a condenser) and heated with a heat gun under N₂. Then, dry THF (14 mL) was added under N₂ and heated while stirring. Afterwards, bromocyclopropane (1.7 mL, 21 mmol) was added portion wise with occasional heating with a heat gun under N₂ until the reaction was self-sustaining (reaction mixture went from orange to colourless). Once the magnesium turnings were consumed, 5-bromo-N-methoxy-N-methylthiophene-3-carboxamide (80%, 5.53 g, 17.7 mmol) was added dropwise under N2 while stirring. The reaction mixture was stirred overnight at 54 °C under N₂ and then quenched with the addition of HCI (2 M) at 0 °C. Then, water (100 mL) was added, the aqueous phase extracted with ethyl acetate (2 × 100 mL), the organic phase washed with water (100 mL), dried with anhydrous Na₂SO₄, filtered and evaporated to dryness. The resulting orange oil (5.25 g) was subjected to flash column chromatography (Aldrich silica gel 60 Å 230-400 mesh 40- 63 µm; eluent: 10% ethyl acetate in hexanes and progressively to 50% ethyl acetate in hexanes) giving the target product as a yellow oil (0.48 g, 12%).

The compound 17 was synthesized by the methods disclosed in W. L. Cody, D. D. Holsworth, N. A. Powell, M. Jalaie, E. Zhang, W. Wang, B. Samas, J. Bryant, R. Ostroski, M. J. Ryan and J. J. Edmunds, Bioorg. Med. Chem., 2005, 13, 59-68.

A compound 39 was synthesized according to the above synthetic scheme by a method disclosed in WO 2008/030226. Specifically, the compound 39 (cyclopropyl(5-(4-((triisopropylsilyl)oxy)phenyl)thiophene-3-yl)methanone) was synthesized by the following method.

A degassed mixture of (5-bromothiophen-3-yl)(cyclopropyl)methanone (9.51 g, 41.1 mmol), (4-((triisopropylsilyl)oxy)phenyl)boronic acid (14.67 g, 49.85 mmol), K2CO3 (6.25 g, 45.2 mmol) and Pd(PPh3)4 (2.44 g, 2.11 mmol) in toluene (490 mL) and ethanol (490 mL) was heated at reflux under N₂ for 16 h. After this time, the mixture was evaporated to dryness, suspended in ethyl acetate (100 mL) and filtered through a plug of celite. The filtrate was reduced, washed with water (2 × 100 mL), dried with anhydrous sodium sulfate, filtered and the solvent removed under reduced pressure affording a brown oil (22.13 g). Purification by flash column chromatography [Aldrich silica gel (60 Å, 40-63 µm), eluent: 10% ethyl acetate in petroleum] followed by recrystallization from petroleum led to the isolation of the corresponding product as a white crystalline solid (10.28 g, 62%).

Compound 39: vmax (neat) 2943, 2864, 1661, 1641, 1603, 1536, 1503, 1446, 1406, 1262, 1173, 1012, 880, 844, 726, 685 cm⁻¹; ¹H NMR (400 MHz, CDCl₃) δH 0.99 - 1.32 (25H, m, CH), 2.49 - 2.55 (1H, m, 2'-H), 6.90 (2H, app. d, J = 8.7 Hz, 3',5'-H), 7.48 (2H, app. d, J = 8.7 Hz, 2',6'-H), 7.65 (1H, d, J = 1.3 Hz, 4-H), 7.99 (1H, d, J = 1.3 Hz, 2-H) ppm; ¹³C{¹H} NMR (100 MHz, CDCl₃) δC 11.4, 12.7, 17.9, 18.0, 120.4, 121.2, 126.6, 127.2, 129.9, 143.7, 145.3, 156.4, 194.8 ppm.

A compound 41 was synthesized according to the above synthetic scheme by the methods disclosed in W. Wu, R. Xu, L. Zhang and S. You, Chem. Sci., 2016, 7, 3427-3431. Specifically, the compound 41 (cyclopropyl(5-(4-hydroxyphenyl)thiophene-3-yl)methanone) was synthesized by the following methods (1) and (2).

Method (1) - Tetrabutylammonium fluoride solution (1 M in THF, 2.8 mL, 2.8 mmol) was added dropwise to a solution of cyclopropyl(5-(4-((triisopropylsilyl)oxy)phenyl)thiophen-3-yl)methanone (1.00 g, 2.50 mmol) in THF (50.0 mL) at 0 °C. The solution was allowed to reach room-temperature and stirred for 83 min. Afterwards, the crude was evaporated to dryness, the residue collected in dichloromethane (50 mL), washed with water (2 × 50 mL), dried with anhydrous Na₂SO₄, filtered and evaporated to dryness giving a pale yellow powder (1.10 g). Flash column chromatography [Aldrich silica gel (60 Å, 40-63 µm), eluent: 10% ethyl acetate in petroleum → 30% ethyl acetate in petroleum] led to the isolation of the corresponding product as a pale yellow powder (0.56 g, 92%).

Method (2) - Tetrabutylammonium fluoride solution (1 M in THF, 23 mL, 23 mmol) was added dropwise to a solution of cyclopropyl(5-(4-((triisopropylsilyl)oxy)phenyl)thiophen-3-yl)methanone (9.13 g, 22.8 mmol) in THF (450 mL) at 0 °C. The solution was allowed to reach room-temperature and stirred for 78 min. Afterwards, the crude was evaporated to dryness, the residue collected in dichloromethane (100 mL), washed with water (2 × 100 mL), dried with anhydrous Na₂SO₄, filtered and evaporated to dryness giving a yellow powder (10.43 g). A pale yellow powder (5.55 g) was obtained by flash column chromatography [Aldrich silica gel (60 Å, 40-63 µm), eluent: 10% ethyl acetate in petroleum → 50% ethyl acetate in petroleum] and later triturated from petroleum and few drops of ethyl acetate giving the corresponding product as a white powder.

Compound 41: vmax (neat) 3366 (br), 1641, 1606, 1449, 1410, 1204, 1167, 1022, 960, 900, 824, 716, 627 cm⁻¹; ¹H NMR (300 MHz, CDCl₃) δH 0.99 - 1.05 (3H, m, CH), 1.21 - 1.26 (2H, m CH), 2.48 - 2.56 (1H, m, 2'-H), 5.78 (1H, s, OH), 7.15 (2H, app. d, J = 8.8 Hz, 3",5"-H), 7.57 (2H, app. d, J = 8.8 Hz, 2",6"-H), 7.67 (1H, d, J = 1.3 Hz, 4-H), 8.01 (1H, d, J = 1.3 Hz, 2-H) ppm; ¹³C{¹H} NMR (100 MHz, CDCl₃) δC 11.5, 18.1, 115.9, 121.3, 126.5, 127.5, 130.0, 143.7, 145.1, 155.8, 195.0 ppm; HRMS (ESI) found [M+H]⁺ = 245.0630 C₁₄H₁₂O₂S requires [M+H]⁺ = 245.0636.

A compound 43 was synthesized according to the above synthetic scheme by the methods disclosed in K. Cantin, A. Lafleur-Lambert, P. Dufour and J. Morin, Eur. J. Org. Chem., 2012, 5335-5349. Specifically, the compound 43 (cyclopropyl(5-(4-(hexyloxy)phenyl)thiophene-3-yl)methanone) was synthesized by the following method.

A mixture of cyclopropyl(5-(4-hydroxyphenyl)thiophen-3-yl)methanone (5.07 g, 20.8 mmol), 1-iodohexane (3.2 mL, 22 mmol) and anhydrous K₂CO₃ (14.40 g, 104.2 mmol) in anhydrous 2-butanone (78 mL) was heated at reflux for 23 h under N2. Then, 1-iodohexane (1.6 mL, 11 mmol) was added and the reaction mixture stirred at reflux 21 h under N₂. Once again, 1-iodohexane (0.8 mL, 5.4 mmol) was added and the reaction mixture stirred at reflux for 3 h under N2. The crude was cooled to room-temperature, poured into water (200 mL), the aqueous phase extracted with CHCl₃ (3 × 100 mL), the combined organics washed with water (2 × 100 mL), dried with anhydrous Na₂SO₄, filtered and evaporated to dryness. The residue (10 g) was triturated from petroleum (100 mL), filtered, rinsed with petroleum (2 × 50 mL) and dried under reduced pressure, giving the target product as a white powder (5.40 g, 79%).

A compound 7 was synthesized according to the above synthetic scheme by the methods disclosed in C. J. Thomas, M. A. Wolak, R. R. Birge and W. J. Lees. J. Org. Chem., 2001, 66, 1914-1918. Specifically, the compound 7 (diethyl 2-((1r,3r,5R,7S)-adamantane-2-ylidene)succinate) was synthesized by the following method.

Diethyl succinate (19.0 mL, 114 mmol) was added to a solution of potassium tert-butoxide (12.90 g, 115.0 mmol) in tert-butyl alcohol (114 mL). After 5 min, 2-adamantanone (17.25 g, 114.8 mmol) was added and the solution stirred at reflux. After 30 min, the crude crashed out of solution, *tert*-butyl alcohol (100 mL) was additionally added and the heterogeneous mixture stirred at reflux for 22 h. Later, the crude as cooled to 0 °C, 23 mL of HCl (50% w/w from 37% conc. HCl) added, the organic residue extracted with ethyl acetate (2 × 100 mL), dried with anhydrous Na₂SO₄, filtered and evaporated to dryness. The resulting residue was suspended in EtOH (470 mL) and conc. HCl (37%, 9.0 mL) slowly added. After 2 days stirring at room temperature, the reaction mixture was quenched with a saturated solution of NaHCO3 (100 mL). Then, the alcohol was removed under reduced pressure, the residue extracted with ethyl acetate (100 mL), washed with water (100 mL), dried with anhydrous Na₂SO₄, filtered and evaporated to dryness. Bulb-to-bulb distillation allowed the removal of diethyl succinate mixed with a sublimated solid impurity (140°C at 2 × 10⁻¹ torr), giving the corresponding product as a dark red oil (21.98 g, 63%).

Compound 7: vmax (neat) 2982, 1733, 1716, 1368, 1282, 1174, 1076, 1028 cm⁻¹; ¹H NMR (400 MHz, CDCl₃) δH 1.09 - 1.34 (6H, m, (CH₃)₂), 1.67 -1.81 (12H, m, CH), 2.69 (1H, s, CH), 3.22 (2H, s, CH₂), 3.54 (1H, s, CH), 3.96 - 4.04 (4H, m, (CH₂)₂) ppm; ¹³C NMR (100 MHz, CDCl₃) δC 14.0, 14.1, 27.5, 34.4, 34.5, 34.9, 36.5, 38.9, 39.1, 60.0, 60.4, 114.3, 162.3, 168.3, 171.3 ppm; HRMS (ESI) found [M+Na]⁺ = 329.1726 C₁₈H₂₆O₄ requires [M+Na]⁺ = 329.1729.

A compound 2 was synthesized according to the above synthetic scheme by the following method.

A solution of diethyl 2-(adamantan-2-ylidene)pentanedioate (2.06 g, 6.70 mmol) in anhydrous toluene (25 mL) was added dropwise to a suspension of NaH [60% dispersion in mineral oil] (0.27 g, 6.72 mmol) in anhydrous toluene (25 mL) at room-temperature under N₂. The suspension was heated to 60°C, one drop of ethanol added. Then, cyclopropyl(5-(4-(hexyloxy)phenyl)thiophen-3-yl)methanone (2.00 g, 6.09 mmol) in anhydrous toluene (25 mL) was added dropwise at 60°C, one drop of ethanol added and the reaction mixture left stirring at 60 °C for 3 days under N₂. The crude was cooled to room-temperature, slowly poured into ice (200 mL) while stirring and then acidified (pH = 1-2) with the addition of HCI (2 M). The phases were separated, the aqueous phase extracted with ethyl acetate (3 × 100 mL), the combined organics washed with water (200 mL), dried with anhydrous Na2SO4, filtered and evaporated to dryness, affording a dark orange oil. The stereoisomeric mixture of 2-(adamantan-2-ylidene)-4-cyclopropyl-3-(ethoxycarbonyl)-4-(5-(4-(hexyloxy)phenyl)thiophen-3-yl)but-3-enoic acids was refluxed for 21 h in a 10% KOH ethanolic solution (74.0 mL). Then, the crude was evaporated to dryness, suspended in water (200 mL), the aqueous phase washed with ethyl acetate (2 × 100 mL) and then the aqueous phase acidified (pH = 1-2) with the addition of HCl (2 M). The aqueous phase was extracted with ethyl acetate (2 × 100 mL), dried with anhydrous Na₂SO₄, filtered and evaporated to dryness giving a red foam (3.13 g). The latter was dissolved in acetic anhydride (50 mL) and heated at reflux for 6 min. The reaction mixture was cooled to room-temperature and the acetic anhydride evaporated off affording a brown oil. Purification by flash column chromatography (Aldrich silica gel 60 Å 230-400 mesh 40-63 µm; eluent: 10% ethyl acetate in petroleum) afforded the Z-fulgide (compound 48) (0.32 g, 10%) and E-fulgide (compound 49) (0.47 g, 14%).

A stereoisomeric mixture of 3- (adamantan-2-ylidene)-4-(cyclopropyl(5-(4-(hexyloxy)phenyl)thiophen-3-yl)methylene)dihydrofuran-2,5-diones (2.36 g, 4.35 mmol) was stirred in degassed toluene (38 mL) under UV irradiation (2 W, 365 nm) under N₂ at room-temperature for 4 days. After this time, the crude was evaporated to dryness. The resulting oil was subjected to flash column chromatography two times: 1st - Aldrich silica gel 60 Å 230-400 mesh 40-63 µm; eluent: 10% ethyl acetate in petroleum, 2nd - Aldrich silica gel 60 Å 230-400 mesh 40-63 µm; eluent: 2% ethyl acetate in toluene. The target product was isolated as an oil that solidified as a yellow powder upon reduced pressured (0.15 g, 6%).

Compound 2: mp = 162-165°C; vmax (neat) 2916, 2854, 1810, 1754, 1607, 1503, 1227, 1176, 1022, 922, 824 cm⁻¹; ¹H NMR (400 MHz, CDCl₃) δH 0.90 - 1.02 (4H, m, CH), 1.17 - 1.30 (4H, m, CH), 1.33 - 1.37 (4H, m, CH), 1.44 - 1.51 (2H, m, CH), 1.62 - 1.83 (8H, m, CH), 1.90 - 1.99 (3H, m, CH), 2.33 (1H, s, CH), 2.53 (1H, s, CH), 2.85 - 2.78 (1H, m, CH), 3.09 (1H, d, J = 14 Hz, CH), 3.34 (1H, d, J = 14 Hz, CH) 3.97 - 4.00 (3H, m, hexyl, 3-H), 6.92 (2H, app. d, J = 8.8 Hz, 3',5'-H), 7.12 (1H, s, 7-H), 7.48 (2H, app. d, J = 8.78 Hz, 2',6'-H) ppm; ¹³C{¹H} NMR (100 MHz, CDCl₃) δC 9.1, 9.5, 12.6, 14.1, 22.6, 25.7, 26.8, 27.6, 29.2, 31.6, 31.8, 33.1, 33.9, 34.6, 36.5, 38.1, 39.2, 51.9, 54.9, 68.2, 115.0, 117.3, 119.0, 125.5, 127.1, 136.6, 142.4, 152.9, 153.8, 159.3, 162.4, 171.3 ppm; HRMS (ESI) found [M+H]⁺ = 543.2566 C₃₄H₃₈O₄S requires [M+H]⁺ = 543.2569.

### Evaluation of Photochromic Performance

The photochromic performance of the compound 2 was evaluated by the method described about Example 1.

### Comparable Examples 1 to 4

The photochromic performances of the following comparative compounds 1 to 4 were evaluated by the method described about Example 1.

Regarding comparative compounds 1 and 2, the color fading speeds were also evaluated by the method described about Example 1.

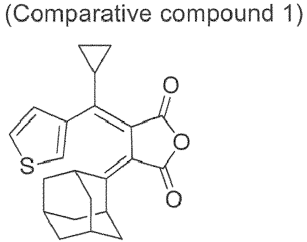

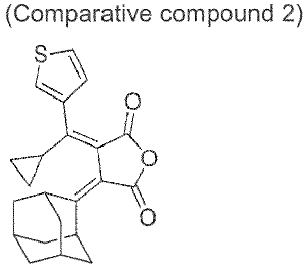

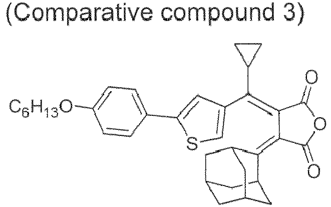

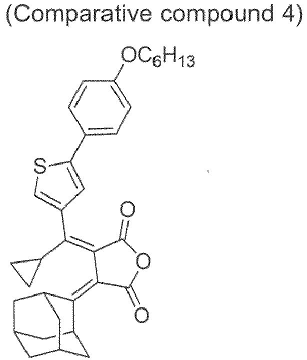

Table 2 shows the results of the above evaluations.

**Table 2**

| | Compound | Photochromic performance (Dynamic range) | Color fading speed (%s⁻¹) |
|---|---|---|---|
| Example 1 | Compound 1 | 35.3 | 0.47 |
| Example 2 | Compound 2 | 33.5 | - |
| Comparative Example 1 | Comparative compound 1 | 5.8 | 0.08 |
| Comparative Example 2 | Comparative compound 2 | 4.8 | 0.06 |
| Comparative Example 3 | Comparative compound 3 | 7.5 | - |
| Comparative Example 4 | Comparative compound 4 | 7.2 | - |

Compounds 1 and 2, which were evaluated in Examples 1 and 2, are compounds represented by the general formula (1). These compounds have structures of a colorless body with ring-closed structures.

In contrast, Comparative compounds 1 to 4, which were evaluated in Comparative Examples 1 to 4, have structures of a colorless body with ring-opened structures.

A comparison between the evaluation results of Examples in 1 and 2 and the evaluation results of Comparative Examples 1 to 4 in Table 2 reveals that compounds represented by the general formula (1), which have structures of a colorless body with ring-closed structures, are excellent in photochromic performance and furthermore, can fade colors at a faster fading speed after irradiation with light.

Finally, the aspects described above are summarized as follows.

According to one aspect, a photochromic compound represented by the general formula (1) is provided.

The photochromic compound represented by the general formula (1) can show excellent photochromic performance. Also, in one embodiment, the photochromic compound represented by the general formula (1) can be a photochromic compound that shows fast fading speed after irradiation with light.

In one embodiment, X in the general formula (1) may denote an oxygen atom.

According to one aspect, a photochromic article containing the photochromic compound described above is provided.

In one embodiment, the photochromic compound can at least have a substrate and a photochromic layer that contains the photochromic compound described above.

In one embodiment, the photochromic article can be a spectacle lens.

In one embodiment, the photochromic article can be a goggle lens.

In one embodiment, the photochromic article can be a visor part of a sun visor.

In one embodiment, the photochromic article can be a shield member of a helmet.

According to one aspect, eyeglasses with the spectacle lens described above are provided.

Various aspects and embodiments described in the present description can be combined as any combination of two or more of these.

The embodiments disclosed here are to be understood as examples and do not restrict the scope of the invention in the entire points. The scope of the invention is determined not by the above description but by the claims and is intended to include the interpretations that are equivalent to the claims and all modifications within the scope of claims.

One aspect of the present invention can be useful in the technical fields of eyeglasses, goggles, sun visors, helmets, and the like.

## Claims

1. A photochromic compound represented by the following general formula (1): 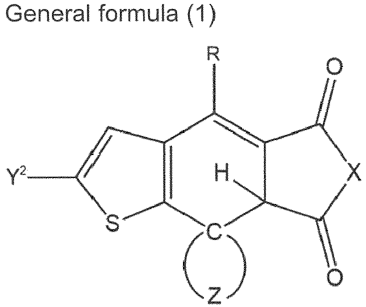
wherein, in the general formula (1),
X denotes an oxygen atom, or a nitrogen atom unsubstituted or substituted by a substituent selected from the following Y¹ group:
Y¹ group: -R¹, -A¹(B¹)ₗ(A²)ₘ(B²)ₙR², -A³A⁴, -A⁵R³
R¹ denotes a cyano group, an alkyl group or an aryl group which may each be substituted,
R² denotes an alkyl group, a naphthyl group, or a naphthyl alkyl group which may each be substituted,
R³ denotes a halogen atom, a cyano group, or a nitro group,
A¹, A², A³, and A⁵ each independently denote an alkylene group, an alkylidene group, a cycloalkylene group, or an alkylcycloalkane-diyl group which may each be substituted,
A⁴ denotes a naphthyl group which may be substituted,
B¹ and B² each independently denote a divalent group selected from the following qroup: 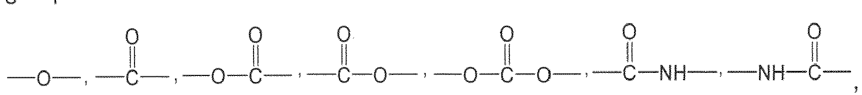
l, m, and n are each independently 0 or 1, provided that n is 0 when m is 0,
Y² denotes a hydrogen atom, or a substituent selected from the above Y¹ group, R denotes a hydrogen atom, a trifluoromethyl group, or a cyclopropyl group which may be substituted, and 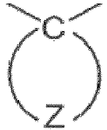
denotes a norbornylidene group, a bicyclo[3.3.1]nonylidene group, or an adamantylidene group which may each be substituted.

2. The photochromic compound according to claim 1,
wherein X in the general formula (1) denotes an oxygen atom.

3. A photochromic article comprising the photochromic compound according to claim 1 or 2.

4. The photochromic article according to claim 3,
which comprises at least:
a substrate; and
a photochromic layer containing the photochromic compound.

5. The photochromic article according to claim 3 or 4,
which is a spectacle lens.

6. The photochromic article according to claim 3 or 4,
which is a goggle lens.

7. The photochromic article according to claim 3 or 4,
which Is a visor part of a sun visor.

8. The photochromic article according to claim 3 or 4,
which is a shield member of a helmet.

9. Eyeglasses comprising the spectacle lens according to claim 5.
